# FASCICULE DE BREVET EUROPEEN

(11) **EP 2 773 608 B1**
(45) Date de publication et mention de la délivrance du brevet: **21.10.2015**
(21) Numéro de dépôt: 12794403.1
(22) Date de dépôt: 30.10.2012
(51) Int. Cl.: C07C 67/08

(54) **PROCÉDÉ DE PRODUCTION D'ACRYLATE DE 2-OCTYLE PAR ESTÉRIFICATION DIRECTE**
VERFAHREN ZUR HERSTELLUNG VON 2-OCTYLACRYLAT DURCH DIREKTE VERESTERUNG
METHOD FOR PRODUCING 2-OCTYL ACRYLATE BY DIRECT ESTERIFICATION

(30) Priorité: 04.11.2011 FR 1159983; 05.07.2012 FR 1256457
(43) Date de publication de la demande: 10.09.2014
(73) Titulaire: Arkema France, 92700 Colombes (FR)
(72) Inventeur: RIONDEL, Alain, 77178 Saint Pathus (FR); GRAIRE, Coralie, F-57890 Porcelette (FR); LEVRAY, André, 57890 Porcelette (FR); LINEMANN, Reinhard, F-57200 Sarreguemines (FR)
(74) Mandataire: Bonnel, Claudine
(86) Numéro de dépôt international: PCT/FR2012/052502
(87) Numéro de publication internationale: WO 2013/064775

(56) Documents cités:
- CN-A- 1 298 863
- US-A- 6 072 076
- US-A1- 2008 015 384

## Description

### DOMAINE DE L'INVENTION

La présente invention concerne la production d'acrylate de 2-octyle selon un procédé en continu par estérification directe.

### ARRIERE-PLAN TECHNIQUE

Il est connu de produire des esters acryliques en mettant en oeuvre une réaction d'estérification entre un alcool et l'acide acrylique. Cette réaction est une réaction catalysée équilibrée avec génération d'eau :

CH₂=CH-COOH + R-OH ⇔ CH₂=CH-COOR + H₂O

Il est nécessaire d'éliminer l'eau produite au cours de la réaction pour déplacer l'équilibre dans le sens de la production de l'ester acrylique.

Cette réaction s'accompagne généralement de réactions secondaires produisant des impuretés qu'il est nécessaire d'éliminer en vue d'obtenir l'ester acrylique avec une pureté élevée satisfaisant aux exigences techniques liées à son utilisation finale en tant que monomère pour fabriquer des polymères utilisables dans de nombreux domaines d'application.

Par ailleurs, pour des raisons économiques évidentes, les produits valorisables présents dans le mélange brut réactionnel, notamment les réactifs non réagis et le catalyseur, sont dans la mesure du possible recyclés au sein du procédé.

A ces fins, on procède généralement à un processus de séparation/purification comportant un ensemble de distillations, d'extractions, et/ou décantations, qui est à la fois relativement complexe à mettre en oeuvre, notamment du fait de la présence de mélanges azéotropes, et coûteux sur le plan énergétique.

Le document US 6,072,076 décrit un procédé de production de (méth)acrylates d'alkyle par estérification de l'acide (méth)acrylique avec un alcool présentant une longueur de chaine allant de 1 à 8 atomes de carbone, en présence d'un catalyseur acide. Dans ce procédé, l'équilibre de la réaction est déplacé en prélevant un mélange comprenant de l'eau et de l'alcool dans une unité de rectification III en tête de la zone de réaction. Le mélange aqueux, après condensation, est traité dans un séparateur 17 afin de purifier l'alcool et le recycler à la réaction par l'intermédiaire de la colonne de rectification III, la phase aqueuse séparée étant partiellement renvoyée à l'unité de rectification III.

Dans ce procédé, le mélange réactionnel issu de la zone de réaction est envoyé dans une unité de rectification I dans laquelle le mélange est séparé en un produit (I) comprenant l'ester recherché, l'alcool et l'acide résiduels, et un produit (II) comprenant le catalyseur ; le produit (I) est soumis à une seconde unité de rectification Il dans laquelle l'ester recherché est séparé des produits alcool et acide résiduels qui sont recyclés à la zone de réaction, l'ester purifié étant soutiré latéralement.

Ce procédé est illustré avec la fabrication de l'acrylate de 2-éthyl hexyle à partir de l'alcool 2-éthyl hexanol, réalisée à l'aide de deux réacteurs 5 et 6 placés en série et opérant à des niveaux thermiques différents. La colonne de rectification III dont le reflux liquide 13 contient de l'eau partiellement recyclée surmonte le premier réacteur. Un mélange brut réactionnel contenant 70% d'acrylate de 2-éthyl hexyle est issu de ce premier réacteur 5. La réaction est poursuivie dans le second réacteur 6 avec soutirage en continu de l'eau produite par la réaction à l'aide de l'unité de rectification III pour atteindre une teneur finale de 82% en acrylate de 2-éthyl hexyle.

Dans le procédé décrit dans le document US 6,072,076, du fait de l'introduction d'eau recyclée dans la colonne de rectification, l'élimination de l'eau générée par la réaction n'est pas complète pour déplacer l'équilibre de la réaction. Il s'avère nécessaire alors de mettre en oeuvre un second réacteur pour atteindre des rendements satisfaisants.

Le procédé décrit dans le document US 6,072,076 n'est pas applicable à la fabrication de l'acrylate de 2-octyle par réaction d'estérification de l'acide acrylique et du 2-octanol. En effet, le 2-octanol est un alcool secondaire beaucoup plus sensible qu'un alcool primaire tel que le 2-éthyl hexanol, à une réaction de déshydratation en présence de catalyseur acide, entraînant la formation d'octènes et d'eau. Cette formation d'eau, cumulée avec la réintroduction dans le système d'au moins une partie de la phase aqueuse générée par la réaction d'estérification, risque de rétrograder plus facilement par hydrolyse l'acrylate de 2-octyle recherché en 2-octanol et acide acrylique. Par ailleurs, selon ce document, la purification du mélange brut réactionnel comportant l'acrylate recherché et de l'alcool résiduel est effectuée par distillation dans l'unité de rectification I avec un temps de séjour important en présence du catalyseur acide. Dans le cas d'une synthèse avec le 2-octanol, cette distillation risque également de générer des octènes et de l'eau et de dégrader au moins partiellement l'acrylate de 2-octyle formé.

La Société Déposante a cherché à résoudre ces différents problèmes liés à la mise en oeuvre du 2-octanol dans la réaction d'estérification avec l'acide acrylique.

Dans le document US 2008/0087196, il est décrit la synthèse d'acrylate de 2-octyle selon une réaction d'estérification avec le 2-octanol, en présence d'acide p-toluène sulfonique. La réaction est réalisée en milieu solvant pour éliminer l'eau produite sous forme d'un azéotrope toluène/eau Un tel procédé est néanmoins compliqué à mettre en oeuvre à l'échelle industrielle.

Par ailleurs, les documents EP 1027 322 et EP 1 028 936 décrivent un procédé pour éliminer le soufre provenant de l'utilisation d'un catalyseur acide sulfonique, dans un procédé de production d'acrylate, notamment d'acrylate de butyle, d'acrylate de 2-éthyle hexyle ou d'acrylate de polyol. Cependant, il n'est nullement suggéré d'appliquer un tel procédé pour la synthèse d'acrylate de 2-octyle.

Dans le brevet CN 1298863 le catalyseur à base de soufre est aussi séparé avant rafinage d'acrylate d'octyle mais le catalyseur est neutralisé et non recyclé.

Il subsiste donc encore un besoin de disposer d'un procédé de fabrication d'acrylate de 2-octyle présentant une productivité compatible avec une fabrication à l'échelle industrielle et conduisant à un acrylate de 2-octyle répondant aux exigences de pureté liées à son utilisation finale.

La présente invention a pour but la fabrication d'un acrylate de 2-octyle de très haute pureté avec un rendement élevé avec la mise en oeuvre d'un seul réacteur, et incluant le recyclage des produits valorisables tels que, d'une part les réactifs non réagis, et d'autre part le catalyseur acide, notamment un catalyseur acide comportant du soufre, en particulier du type acide sulfonique.

La solution proposée consiste à ne pas réintroduire directement dans la colonne de rectification la phase aqueuse produite par la réaction afin d'optimiser le déplacement d'équilibre de la réaction dans le sens de la production de l'ester, et à séparer le catalyseur acide en vue de son recyclage préalablement à l'ensemble du processus de purification de l'acrylate de 2-octyle recherché.

Selon l'invention, le catalyseur, notamment acide comportant du soufre, est réutilisé dans le processus réactionnel. L'émission de composés organo-soufrés au cours du procédé de production d'acrylate de 2-octyle utilisant un catalyseur de type acide sulfonique, se trouve ainsi réduite, rendant le procédé selon l'invention respectueux de l'environnement.

La présente invention permet en outre de produire un ester acrylique comportant du carbone d'origine renouvelable lié à l'utilisation du 2-octanol, qui est un alcool dérivé de matières végétales.

### RESUME DE L'INVENTION

La présente invention a donc pour objet un procédé de production en continu d'acrylate de 2-octyle par réaction d'acide acrylique et de 2-octanol en présence d'un catalyseur d'estérification de type acide comportant du soufre et d'au moins un inhibiteur de polymérisation, l'eau formée par la réaction d'estérification étant entrainée par distillation dans une colonne surmontant le réacteur sous la forme d'un mélange hétéroazéotropique avec le 2-octanol qui est soumis ensuite, après condensation, à une séparation dans un décanteur pour donner une phase organique supérieure et une phase aqueuse inférieure, le mélange réactionnel contenant le produit de réaction et des sous-produits résiduels étant soumis à un traitement de purification par des moyens de séparation, afin d'obtenir d'une part l'acrylate de 2-octyle pur, d'autre part les composés 2-octanol et acide acrylique non réagis destinés à être recyclés, et aussi le catalyseur destiné à être recyclé, procédé caractérisé en ce que :
- la phase aqueuse issue du décanteur est soutirée en continu pour être éliminée ;
- la phase organique issue du décanteur est recyclée en continu en pied de la colonne de distillation surmontant le réacteur ;
- le traitement de purification du mélange réactionnel comprend les étapes consécutives suivantes (i) la séparation d'un flux riche en catalyseur qui est recyclé au moins en partie à l'étape de réaction, (ii) la séparation d'un flux riche en 2-octanol non réagi qui est recyclé au moins en partie à l'étape de réaction, et (iii) la récupération de l'acrylate de 2-octyle pur recherché.

L'expression « riche » utilisée dans la définition de l'invention est employée avec un sens relatif, afin de distinguer les flux les uns par rapport aux autres. Ainsi, le « flux riche en acrylate de 2-octyle » est un flux comportant davantage d'acrylate de 2-octyle que le « flux riche en 2-octanol» et que le « flux riche en catalyseur et inhibiteur de polymérisation » ; le « flux riche en 2-octanol» est un flux comportant davantage de composé 2-octanol que le « flux riche en acrylate de 2-octyle».

Selon l'invention, le mélange réactionnel contient le produit de réaction (acrylate de 2-octyle) et des sous-produits résiduels (sous-produits lourds de haut point d'ébullition et des oligomères ; les réactifs non réagis, le catalyseur et des inhibiteurs de polymérisation).

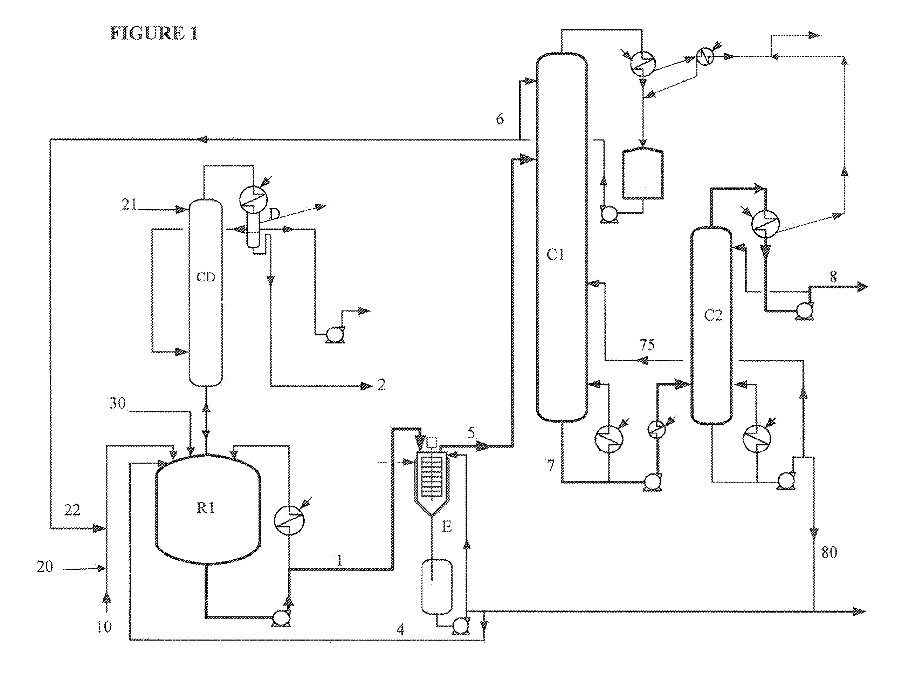

L'invention est maintenant décrite plus en détail et de façon non limitative dans la description qui suit, en référence aux figures 1, 2 et 3 qui illustrent de manière schématique une installation permettant de mettre en oeuvre trois modes de réalisation du procédé selon l'invention :
- Figure 1 : illustre un premier mode de réalisation, basé sur la séparation préalable d'un flux riche en catalyseur à l'aide d'un évaporateur à film effectuée sur le mélange réactionnel, puis la purification de l'acrylate de 2-octyle à partir de deux colonnes de distillation.

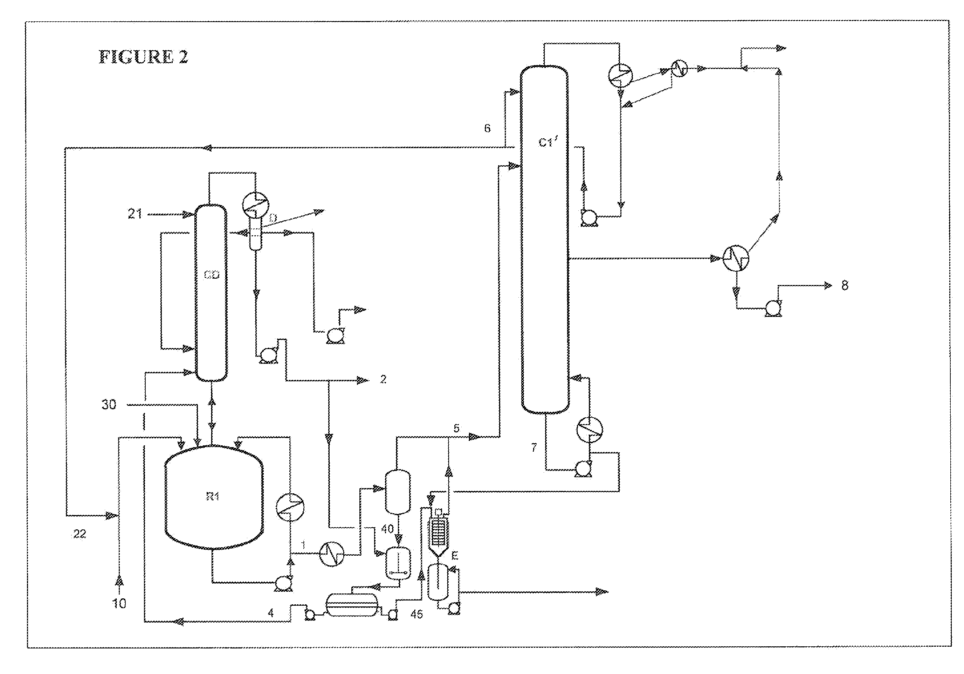

- Figure 2 : illustre un second mode de réalisation qui met en oeuvre la séparation d'un flux riche en produit de réaction par évaporation partielle du mélange réactionnel, suivie, d'une part de la purification du produit de réaction à l'aide d'une colonne de distillation et soutirage latéral de l'acrylate de 2-octyle pur, et d'autre part, d'une extraction à l'eau du catalyseur acide présent dans le résidu de mélange réactionnel et de son recyclage à la réaction.

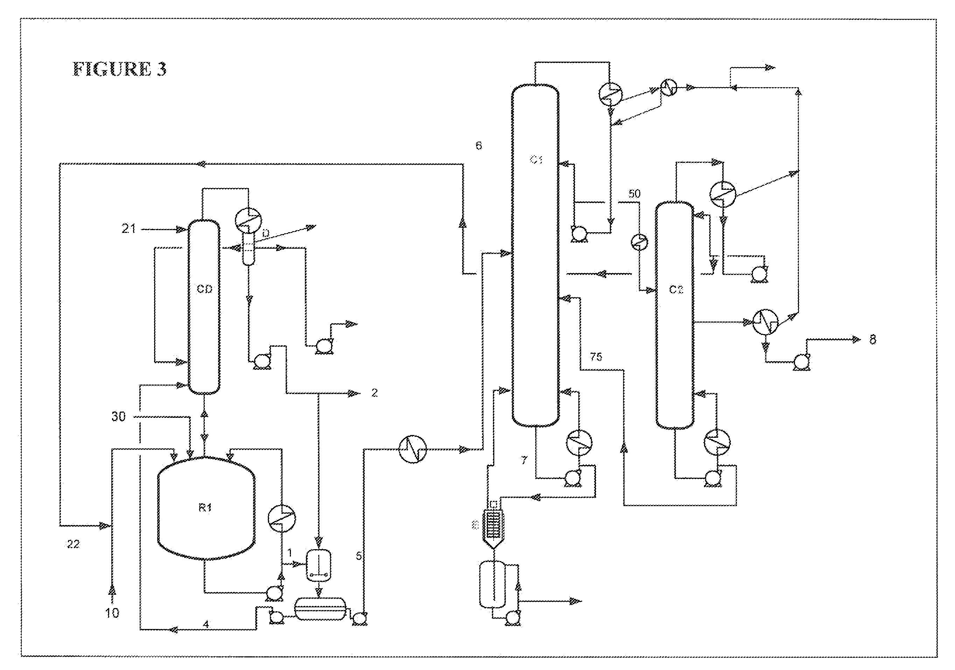

- Figure 3 : illustre un troisième mode de réalisation, qui met en oeuvre une extraction à l'eau du catalyseur présent dans le mélange réactionnel et son recyclage à la réaction, suivie de la purification du mélange réactionnel exempt de catalyseur à l'aide de deux colonnes de distillation avec soutirage latéral de l'acrylate de 2-octyle pur.

### DESCRIPTION DETAILLEE

L'un des objectifs de l'invention est d'utiliser des matières premières d'origine naturelle et renouvelable, c'est-à-dire bio ressourcées.

Le 2-octanol utilisé dans le procédé selon l'invention est un alcool d'origine renouvelable, en particulier il peut être obtenu par traitement alcalin de l'acide ricinoléïque dérivé de l'huile de ricin.

L'acide acrylique mis en oeuvre comme matière première dans le procédé selon l'invention est essentiellement produit sur le plan industriel à partir du propylène.

Mais, indépendamment de la mise en oeuvre de l'alcool d'origine renouvelable, l'invention s'étend à l'utilisation, lors de l'estérification, d'acide acrylique d'origine renouvelable, pouvant être en particulier obtenu à partir de glycérol, selon un procédé comportant une première étape de déshydratation du glycérol en acroléine suivie d'une étape d'oxydation en phase gaz de l'acroléine ainsi obtenue ; ou obtenu par déshydratation des acides 2-hydroxypropionique (acide lactique) ou 3-hydroxypropionique et de leurs esters.

La réaction d'estérification est réalisée à partir des réactifs dans un rapport molaire 2-octanol/acide acrylique pouvant aller de 0,5 à 3, en particulier compris entre 1 et 1,5, à une température généralement comprise entre 80°C et 130°C, de préférence entre 90°C et 120°C, à pression normale ou sous pression réduite, par exemple comprise entre 50 et 200 mbars et la pression atmosphérique.

On utilise un catalyseur d'estérification acide, généralement un composé acide comportant du soufre, tel qu'un acide organique sulfonique, comme l'acide méthane sulfonique, l'acide para-toluène sulfonique, l'acide benzène sulfonique, l'acide dodécylsulfonique, l'acide xylène sulfonique, ou leurs mélanges, ou l'acide sulfurique. De préférence, on utilise l'acide méthane sulfonique comme catalyseur d'estérification. Le catalyseur est avantageusement introduit à raison de 5.10⁻⁴ mole à 5.10⁻² mole par mole de 2-octanol.

La réaction est effectuée en présence d'un ou plusieurs inhibiteurs de polymérisation qui sont introduits dans le réacteur, à raison de 500 à 5000 ppm par rapport au mélange brut réactionnel. Comme inhibiteurs de polymérisation utilisables, on peut citer par exemple la phénothiazine, l'hydroquinone, l'éther monométhylique d'hydroquinone, le diterbutyl para-crésol (BHT), la paraphénylène diamine, le TEMPO (2,2,6,6-tétraméthyl-1-piperidinyloxy), le di-tertiobutylcatéchol, ou les dérivés du TEMPO, tel que le OH-TEMPO, seuls ou leurs mélanges en toutes proportions. Un ajout supplémentaire d'inhibiteur de polymérisation est généralement effectué au niveau du traitement ultérieur de purification.

Conformément aux schémas représentés sur les figures 1, 2 et 3, la réaction d'estérification est réalisée dans un réacteur R1, surmonté d'une colonne à distiller CD.

Le réacteur est un réacteur parfaitement agité, chauffé par un échangeur externe. Il est surmonté d'une colonne à distiller CD à garnissage ordonné ou à plateaux comprenant par exemple une dizaine de plateaux théoriques. L'ensemble fonctionne sous une pression comprise entre 50 à 350 mbars et la pression atmosphérique.

Dans le réacteur, on introduit directement l'acide acrylique par la ligne 10 et le catalyseur et les inhibiteurs de polymérisation par la ligne 30. Le 2-octanol est introduit en partie directement dans le réacteur par la ligne 20, et en partie en tête de la colonne CD par la ligne 21 pour assurer le reflux de la colonne. Par la ligne 22, il peut être introduit un flux recyclé riche en 2-octanol provenant des étapes de purification ultérieures.

Pendant la réaction, on distille un mélange contenant majoritairement l'hétéroazéotrope 2-octanol / eau et minoritairement de l'acide acrylique, qui se sépare, après condensation, en deux phases dans le décanteur D. A cet effet, l'installation comprend un condenseur alimenté par de l'eau à 25°C, et un décanteur recueillant l'hétéroazéotrope condensé, muni d'un système permettant éventuellement de maintenir un niveau constant de phase aqueuse inférieure par soutirage automatique de l'eau de réaction au fur et à mesure de sa formation grâce à l'ouverture d'une électrovanne. Un système de vide régulé permet de travailler sous pression réduite.

La phase organique supérieure 3 riche en 2-octanol et comprenant minoritairement de l'acide acrylique, est renvoyée par débordement en pied de la colonne CD.

La phase aqueuse 2, est soutirée en continu et n'est pas réintroduite dans la colonne de distillation CD. Le soutirage continu de cette phase aqueuse permet de déplacer l'équilibre de la réaction d'estérification en permanence vers la formation de l'acrylate de 2-octyle et donc d'améliorer sa sélectivité.

Selon un mode de réalisation optionnel, la phase aqueuse peut être soumise à une distillation permettant de récupérer le 2-octanol et l'acide acrylique présent en faible teneur, avant son traitement biologique et rejet.

Après réaction avec un temps de séjour généralement compris entre 2 et 5 heures, le mélange réactionnel 1 comprend entre 60 et 90% en poids de produit de réaction (acrylate de 2-octyle), avec du 2-octanol et acide acrylique non réagis, le catalyseur et les inhibiteurs de polymérisation, ainsi que des sous-produits lourds.

Selon l'invention, le mélange réactionnel est soumis à un traitement de purification visant à récupérer l'acrylate de 2-octyle d'une grande pureté, et à recycler les produits valorisables et éviter le rejet de flux polluants, tout en minimisant le bilan énergétique de l'installation.

Le traitement de purification peut être effectué selon différentes variantes.

Selon un premier mode de réalisation, illustré sur la figure 1, le traitement de purification du mélange réactionnel 1 comprend les sous-étapes suivantes :
- une première étape de séparation du catalyseur à l'aide d'un évaporateur à film E, à l'issue de laquelle on sépare en pied un flux 4 riche en catalyseur et inhibiteurs de polymérisation, ledit flux 4 étant recyclé au moins en partie à la réaction, et en tête un flux 5 riche en acrylate de 2-octyle ;
- le flux 5 riche en acrylate de 2-octyle est envoyé sur une première colonne de distillation C1 permettant de récupérer en tête un flux 6 riche en 2-octanol qui est recyclé à la réaction, et en pied un flux 7 riche en acrylate de 2-octyle qui est envoyé sur une seconde colonne de distillation C2 permettant de séparer en tête 8 l'acrylate de 2-octyle pur, et en pied une fraction contenant des impuretés lourdes et des inhibiteurs de polymérisation qui est, soit recyclée vers la première colonne de distillation, soit éliminée.

Comme évaporateur à film, on peut notamment utiliser un évaporateur à film tombant ou un évaporateur à film raclé. Ce type d'évaporateur présente l'avantage d'avoir un temps de séjour réduit ce qui limite la formation de composés lourds supplémentaires en aval de la section réaction. Cet évaporateur se compose essentiellement d'une partie cylindrique chauffée par double enveloppe, d'une partie supérieure servant à la séparation des vapeurs et d'un rotor tournant à grande vitesse. Le flux de mélange réactionnel à traiter est étalé sur toute la surface de chauffe sous forme d'un film à grande turbulence. Les vapeurs qui se forment montent à contre-courant vers le haut de l'appareil. Les produits non évaporés, essentiellement le catalyseur et les inhibiteurs de polymérisation, atteignent la partie inférieure de l'évaporateur et sont évacués sous forme d'un flux 4. Le flux gazeux 5, en tête de l'évaporateur, constitue l'alimentation de la première colonne C1 du train de purification. L'évaporateur à film fonctionne dans les conditions de fonctionnement de la colonne C1.

Le flux 4 contenant pratiquement la totalité du catalyseur et des inhibiteurs de polymérisation est avantageusement recyclé à la réaction, de préférence après une purge des impuretés lourdes générées au cours de la réaction, notamment les adduits de Michael résultant de l'addition du 2-octanol sur l'acrylate de 2-octyle, ou l'ester du dimère de l'acide acrylique.

Le flux 5 riche en acrylate de 2-octyle est envoyé dans une première colonne de distillation C1 pour récupérer en tête un flux 6 riche en 2-octanol. La colonne C1 est en général une colonne à garnissage ordonné ou à plateaux comportant de l'ordre de 15 plateaux théoriques et opérant sous pression réduite, par exemple entre 20 et 80 mbars, et elle est chauffée par un échangeur externe type thermo-siphon ou à recirculation forcée. En tête de cette colonne C1, on introduit en général un inhibiteur de polymérisation en solution dans le 2-octanol.

Le flux 6, contient du 2-octanol majoritairement, mais aussi de l'acide acrylique résiduel et une faible teneur d'acrylate de 2-octyle. Il est avantageusement recyclé à la réaction par la ligne 22. L'élimination préalable du catalyseur acide à l'aide de l'évaporateur à film, minimise la formation d'octènes dans ce flux 6 au cours de la distillation des composés légers dans la colonne C1.

La purification du flux 7 de pied de la colonne C1, riche en acrylate de 2-octyle est poursuivie à l'aide d'une seconde colonne de distillation C2 permettant d'éliminer en pied les impuretés encore présentes et les inhibiteurs de polymérisation qui sont en partie recyclés par la ligne 75 dans la partie supérieure de la colonne C1 ou en partie envoyés par la ligne 80 au niveau d'une unité de traitement. La colonne C2 est une colonne à garnissage ordonné ou à-plateaux-comportant de l'ordre de 5 à 10 plateaux théoriques qui fonctionne par exemple sous 10 mbars en tête de colonne et chauffée par un échangeur externe type thermo-siphon ou à recirculation forcée. En tête de cette colonne C2, on introduit en général un inhibiteur de polymérisation en solution dans l'acrylate de 2-octyle.

L'acrylate de 2-octyle pur est récupéré en tête de la colonne C2 (flux 8).

Selon un second mode de réalisation, illustré sur la figure 2, le traitement de purification du mélange réactionnel 1 comprend les sous-étapes suivantes :
- l'évaporation flash du mélange réactionnel 1 conduisant à la séparation d'un flux 5 riche en produit de réaction et d'un résidu 40 de mélange réactionnel ;
- la mise en contact sous agitation du résidu 40 de mélange réactionnel avec de l'eau ou un flux aqueux pour former un système biphasique constitué d'une phase aqueuse 4 comprenant le catalyseur, et d'une phase organique 45 riche en sous-produits lourds issus de la réaction ;
- la séparation de ladite phase aqueuse 4 et son recyclage à la réaction ;
- la séparation de ladite phase organique 45 qui est envoyée à un évaporateur à film E permettant de séparer un flux de produits valorisables, tels que produit de réaction et réactifs non réagis, qui est mélangé au flux 5 ;
- le flux 5 riche en acrylate de 2-octyle est envoyé sur une colonne de distillation C1' permettant de récupérer :
   o en tête un flux 6 riche en 2-octanol qui est recyclé à la réaction,
   o l'acrylate de 2-octyle pur via un soutirage latéral, et
   o en pied un flux 7 riche en sous-produits lourds et contenant de l'acrylate de 2-octyle qui est envoyé sur l'évaporateur à film E à partir duquel une fraction contenant les sous-produits lourds et débarrassée de composés valorisables, est éliminée.

Selon ce second mode de réalisation, le catalyseur acide est extrait du mélange réactionnel débarrassé de l'essentiel du produit de réaction (flux 40).

La séparation du flux 5 riche en produit de réaction est réalisée à l'aide d'une évaporation flash comprenant le chauffage du mélange réactionnel suivie d'une détente à la pression de fonctionnement de la colonne C1' vers laquelle est envoyé ledit flux 5. On peut notamment chauffer le milieu réactionnel à une température de l'ordre de 180°C sous une pression de 3 bars, puis effectuer une détente à environ 65 mbar dans un pot flash à une température de l'ordre de 110°C.

Le résidu 40 de mélange réactionnel est mis en contact et mélangé dans un extracteur avec de l'eau ou un flux aqueux. Avantageusement, on utilise comme flux aqueux d'extraction, la phase aqueuse issue du décanteur D de séparation du mélange hétéroazéotropique généré par la réaction. Le mélange est réalisé par tout moyen de mélange à une vitesse de mélange suffisante pour permettre le transfert de masse requis pour l'extraction du composé acide du résidu, et la formation d'un système biphasique aqueux/organique.

La phase aqueuse décantée 4, comprenant l'essentiel du catalyseur, est recyclée dans le réacteur R1, ou de préférence dans la partie basse de la colonne CD, après décantation.

La phase organique 45, pouvant comporter des produits valorisables tels que produit de réaction, inhibiteurs de polymérisation et réactifs résiduels, est envoyée sur un évaporateur à film E permettant de récupérer un flux de produits valorisables, qui est mélangé au flux 5 avant la purification réalisée au niveau de la colonne C1'.

La colonne C1' est en général une colonne comportant de 15 à 20 plateaux théoriques fonctionnant sous 20 mmHg.

La colonne C1' fonctionne comme une colonne d'équeutage/étêtage, c'est-à-dire permet de séparer en tête un flux 6 riche en composé léger tel que le 2-octanol non réagi, et en pied un flux 7 riche en sous-produits lourds, ledit flux 7 étant éliminé après passage sur l'évaporateur à film E.

L'acrylate de 2-octyle pur est récupéré directement au niveau de cette colonne C1' via un soutirage latéral, en phase liquide ou en phase gazeuse, de préférence en phase gazeuse, en général à un niveau intermédiaire situé dans la partie basse de la colonne, en particulier entre les plateaux théoriques 12 et 18.

Ce mode de réalisation présente l'avantage de ne mettre en oeuvre qu'une seule colonne de distillation C1'.

Selon un troisième mode de réalisation, illustré sur la figure 3, le traitement de purification du mélange réactionnel 1 comprend les sous-étapes suivantes :
- la mise en contact du mélange réactionnel 1 avec de l'eau ou un flux aqueux pour former un système biphasique constitué d'une phase aqueuse 4 comprenant le catalyseur, et d'une phase organique 5 riche en produit de réaction ;
- la séparation de ladite phase aqueuse 4 et son recyclage à la réaction ;
- la séparation de ladite phase organique 5 qui est envoyée sur une première colonne de distillation C1 permettant d'obtenir :
   o en tête un flux 50 riche en produit de réaction ; et
   o en pied un flux 7 riche en sous-produits lourds et contenant de l'acrylate de 2-octyle, ledit flux 7 étant envoyé sur un évaporateur à film E à partir duquel une fraction contenant les sous-produits lourds et débarrassée des composés valorisables recyclés à la colonne C1, est éliminée ;
- le flux 50 riche en produit de réaction est envoyé sur une deuxième colonne de distillation C2 permettant de récupérer :
   o en tête un flux 6 riche en 2-octanol qui est recyclé à la réaction ;
   o l'acrylate de 2-octyle pur via un soutirage latéral ; et
   o en pied un flux riche en acrylate de 2-octyle avec des inhibiteurs de polymérisation, qui est recyclé au moins en partie par la ligne 75 au niveau de la colonne C1.

Comme le second mode de réalisation décrit précédemment, l'extraction du catalyseur à l'eau directement sur le mélange réactionnel, minimise l'introduction de composé acide fort dans le processus de purification et en conséquence la formation d'octènes.

Le mélange réactionnel issu du réacteur R1 est directement mis en contact et mélangé dans un extracteur avec de l'eau, ou un flux aqueux. Avantageusement, on utilise comme flux aqueux d'extraction, la phase aqueuse issue du décanteur D de séparation du mélange hétéroazéotropique généré par la réaction. Le mélange est réalisé par tout moyen de mélange à une vitesse de mélange suffisante pour permettre le transfert de masse requis pour l'extraction du composé acide du résidu, et la formation d'un système biphasique aqueux/organique.

La phase aqueuse décantée 4, comprenant l'essentiel du catalyseur, est recyclée dans le réacteur R1, ou de préférence dans la partie basse de la colonne CD, après décantation.

La purification de la phase organique 5 est effectuée à l'aide d'une première colonne de distillation C1, fonctionnant comme une colonne d'équeutage (séparation des sous-produits lourds) et d'une seconde colonne de distillation C2 fonctionnant comme une colonne d'étêtage.

Les colonnes C1 et C2 sont par exemple des colonnes comportant entre 10 et 20 plateaux théoriques et fonctionnant sous 20 mmHg en tête.

Le fonctionnement de la colonne C2 permet d'obtenir l'acrylate de 2-octyle pur à un niveau intermédiaire situé dans la partie basse de la colonne, en particulier entre les plateaux théoriques 12 et 18.

Le procédé de l'invention selon ses différentes variantes permet de produire un acrylate de 2-octyle de pureté supérieure à 99,5%, voire supérieure à 99,8%.

Les exemples suivants illustrent l'invention sans la limiter.

### PARTIE EXPERIMENTALE

Dans les exemples, les pourcentages sont indiqués en poids sauf indication contraire et les abréviations suivantes ont été utilisées :
AA : acide acrylique
A2OCT : acrylate de 2-octyle
PTZ : phénothiazine
AMS : acide méthane sulfonique

### Exemple 1 (selon l'invention, en référence au premier mode de réalisation illustré par la figure 1)

Dans le réacteur R1, on introduit en continu à l'aide d'un tube plongeant, du 2-octanol, le catalyseur AMS, l'acide acrylique AA et le stabilisant PTZ. Du 2-octanol est introduit aussi en tête de la colonne à distiller CD qui surmonte le réacteur. Ces différents constituants sont dans les proportions massiques 63,9 / 1 / 35 / 0,1.

La réaction d'estérification s'effectue sous pression réduite à 100 mbars à une température de 115°C, avec un temps de séjour de 3h. Au cours de cette étape, l'équilibre de la réaction est déplacé en soutirant en tête de colonne CD l'eau sous forme d'un hétéroazéotrope 2-octanol/eau de composition massique 10/90 qui est soumis après condensation à une séparation de phases dans un décanteur D. La phase aqueuse inférieure A, contenant une faible quantité de 2-octanol, issue de ce décanteur est soutirée en continu et éliminée après avoir récupéré le 2-octanol à l'aide d'une colonne de distillation (non représentée sur le schéma). La phase organique surnageante O issue du décanteur D est renvoyée en continu en pied de la colonne CD.

Le brut réactionnel 1 en sortie du réacteur R1 a la composition massique suivante :
- A2OCT : 85,6%
- 2-Octanol : 11,3%
- AA:1%
- PTZ + AMS : 2,1%

Le flux 1 issu du réacteur R1 est envoyé vers un évaporateur à film E qui sépare en tête un flux 5 débarrassé de l'AMS, et en pied un flux 4 contenant majoritairement le catalyseur AMS et le stabilisant PTZ.

Le flux 4 contient de l'A2OCT, du 2-octanol, de l'AA, de l'AMS avec le stabilisant dans les proportions massiques 79,3 / 4 / 0,1 /16,6. Ce flux 4 est recyclé pour partie majoritaire à la réaction, l'autre partie minoritaire est envoyée vers le traitement des lourds (non représentée sur le schéma).

Le flux 5, débarrassé du catalyseur acide et du stabilisant contient l'A2OCT, le 2-octanol, l'AA dans les proportions massiques 86,9 /12 / 1,1. Ce flux 5 est envoyé vers une première colonne de distillation C1 qui sépare en tête un flux 6 riche en alcool non-réagi, et en pied un flux 7 contenant majoritairement l'A2OCT recherché.

Le flux 6 est constitué de 2-octanol, d'AA non transformé et d'A2OCT dans la composition massique 80,7 / 7,1 / 12,2. Ce flux 6 est recyclé à la réaction d'estérification.

Le flux 7, pied de la colonne C1 est envoyé vers une seconde colonne de distillation C2 qui sépare en tête l'A2OCT pur 8 de composition :
A2OCT : 99,8%
AA: 100 ppm
2-octanol : 0,1%
Autres impuretés : < 0,1%
et en pied des composés lourds qui sont, soit envoyés vers la station de traitement des lourds, soit recyclés dans la partie supérieure de la colonne C1.

### Exemple 2 (comparatif)

Le brut réactionnel 1 est envoyé directement vers la colonne à distiller C1. Dans ce cas, la présence du catalyseur acide favorise la formation d'octènes dans la colonne et le flux 6 qui sort en tête de colonne C1 contient 10% d'octènes. Ce flux n'est donc pas recyclable en l'état à la réaction et nécessite une purification supplémentaire pour éliminer les octènes.

Le flux 7 en pied de la colonne C1 contenant l'A2OCT, le catalyseur AMS et le stabilisant PTZ est envoyé vers la colonne à distiller C2 pour séparer en tête l'A2OCT purifié. Dans ce cas la pureté de l'A2OCT n'est que de 98,5%. Il contient 1% d'octènes et 5000 ppm de 2-octanol. L'acrylate de 2-octyle obtenu n'est pas utilisable comme monomère pour fabriquer des polymères adhésifs à la pression.

### Exemple 3 (selon l'invention, en référence au second mode de réalisation illustré par la figure 2)

Le flux 1 issu du réacteur R1 est préchauffé à 3 bar eff / 180°C dans un échangeur, puis est détendu à 65 mbars abs /110°C dans un pot flash. Le flux de tête 5 du pot flash alimente la colonne de distillation C1'. Le pied du flash est mélangé avec la totalité de l'eau de réaction provenant de la tête de la colonne CD. Après décantation, on récupère une phase aqueuse 4 constituée d'eau et d'AMS, qui est recyclée dans la partie basse de la colonne de distillation surmontant le réacteur, et une phase organique 45 constituée d'A2OCT, de réactifs non convertis et de composés lourds et des stabilisants. Ce flux alimente un évaporateur E fonctionnant sous 65 mbar abs / 135°C. En pied sortent les composés lourds qui sont envoyés pour traitement à l'extérieur de l'atelier, et en tête, on récupère un flux riche en A2OCT qui se mélange au flux 5 pour alimenter la colonne C1'.

La colonne C1' fonctionne sous un vide de 20 mmHg et comporte 17 plateaux théoriques. Elle sépare en tête un flux 6 riche en 2-octanol, en pied un flux 7 riche en A2OCT et stabilisant, et en latéral l'A2OCT pur (flux 8).

En partant d'un mélange réactionnel 1 de composition :
- A2OCT : 65%
- 2-Octanol : 14%
- AA : 9%
- PTZ + AMS : 2%

On obtient les flux de compositions suivantes :
Flux 5 :
   - A2OCT : 68,3%
   - 2-Octanol : 14,2%
   - AA : 9,2%
Flux 6 :
   - A2OCT : 4,9%
   - 2-Octanol : 43,3%
   - AA : 28,1%
   ce flux 6 peut être recyclé à la réaction
Flux 8 :
   - A2OCT : 99,8%
   - 2-Octanol : 0,1%
   - AA : 100 ppm
Autres impuretés : < 0,1%

Le flux 8 est constitué du produit recherché de grande pureté.

### Exemple 4 (selon l'invention, en référence au troisième mode de réalisation illustré par la figure 3)

Le flux 1 issu du réacteur R1 est envoyé vers une section d'extraction du catalyseur constituée d'un mélangeur-décanteur qui fonctionne à 80°C. Dans cette section le catalyseur est extrait du flux 1 par la phase aqueuse A issue du décanteur D

Après décantation, on récupère :
- en phase inférieure, une phase aqueuse qui contient entre 25 et 30% d'AMS et qui est recyclée à la réaction par envoi en partie basse de la colonne CD,
- en phase supérieure, une phase organique 5 qui ne contient plus que 200 ppm d'AMS et qui a la composition massique suivante :
   - A2OCT : 86,5%
   - 2-Octanol : 11,8%
   - AA : 1,5%
   - PTZ + AMS : 0,2%

Ce flux 5 est envoyé vers une colonne C1 qui sépare en tête un mélange 50 de composition :
A2OCT : 87%
2-Octanol : 12%
AA : 1%

Et, en pied un mélange 7 riche en A2OCT et sous-produit lourds qui est envoyé vers un évaporateur E pour récupérer en tête un flux contenant majoritairement de l'A2OCT et du 2-octanol qui est renvoyé vers la colonne C1, et en pied les sous produits lourds qui sont éliminés.

Le mélange 50 est envoyé vers une colonne C2 qui sépare en tête un mélange 6 riche en 2-octanol et en pied un mélange riche en A2OCT qui est recyclé pour partie vers la colonne C1 via la ligne 75.

L'A2OCT pur obtenu en soutirage latéral de la colonne C2 a la pureté suivante :
A2OCT : 99,8%
AA: 100 ppm
2-octanol : 0,1%
Autres impuretés : < 0,1%

## Revendications

1. Procédé de production en continu d'acrylate de 2-octyle par réaction d'acide acrylique et de 2-octanol en présence d'un catalyseur d'estérification de type acide comportant du soufre et d'au moins un inhibiteur de polymérisation, l'eau formée par la réaction d'estérification étant entrainée par distillation dans une colonne surmontant le réacteur sous la forme d'un mélange hétéroazéotropique avec le 2-octanol qui est soumis ensuite, après condensation, à une séparation dans un décanteur pour donner une phase organique supérieure et une phase aqueuse inférieure, le mélange réactionnel contenant le produit de réaction et des sous-produits résiduels étant soumis à un traitement de purification par des moyens de séparation, afin d'obtenir d'une part l'acrylate de 2-octyle pur, d'autre part les composés 2-octanol et acide acrylique non réagis destinés à être recyclés, et aussi le catalyseur destiné à être recyclé, procédé **caractérisé en ce que** :
- la phase aqueuse issue du décanteur est soutirée en continu pour être éliminée ;
- la phase organique issue du décanteur est recyclée en continu en pied de la colonne de distillation surmontant le réacteur ;
- le traitement de purification du mélange réactionnel comprend les étapes consécutives suivantes (i) la séparation d'un flux riche en catalyseur qui est recyclé au moins en partie à l'étape de réaction, (ii) la séparation d'un flux riche en 2-octanol non réagi qui est recyclé au moins en partie à l'étape de réaction, et (iii) la récupération de l'acrylate de 2-octyle pur recherché.

2. Procédé selon revendication 1 **caractérisé en ce que** le traitement de purification du mélange réactionnel 1 comprend les sous-étapes suivantes :
- une première étape de séparation du catalyseur à l'aide d'un évaporateur à film E, à l'issue de laquelle on sépare en pied un flux 4 riche en catalyseur et inhibiteurs de polymérisation, ledit flux 4 étant recyclé au moins en partie à la réaction, et en tête un flux 5 riche en acrylate de 2-octyle ;
- le flux 5 riche en acrylate de 2-octyle est envoyé sur une première colonne de distillation C1 permettant de récupérer en tête un flux 6 riche en 2-octanol qui est recyclé à la réaction, et en pied un flux 7 riche en acrylate de 2-octyle qui est envoyé sur une seconde colonne de distillation C2 permettant de séparer en tête 8 l'acrylate de 2-octyle pur, et en pied une fraction contenant des impuretés lourdes et des inhibiteurs de polymérisation qui est, soit recyclée vers la première colonne de distillation, soit éliminée.

3. Procédé selon la revendication 1 **caractérisé en ce que** le traitement de purification du mélange réactionnel 1 comprend les sous-étapes suivantes :
- l'évaporation flash du mélange réactionnel 1 conduisant à la séparation d'un flux 5 riche en produit de réaction et d'un résidu 40 de mélange réactionnel ;
- la mise en contact sous agitation du résidu 40 de mélange réactionnel avec de l'eau ou un flux aqueux pour former un système biphasique constitué d'une phase aqueuse 4 comprenant le catalyseur, et d'une phase organique 45 riche en sous-produits lourds issus de la réaction ;
- la séparation de ladite phase aqueuse 4 et son recyclage à la réaction ;
- la séparation de ladite phase organique 45 qui est envoyée à un évaporateur à film E permettant de séparer un flux de produits valorisables, tels que produit de réaction et réactifs non réagis, qui est mélangé au flux 5 ;
- le flux 5 riche en acrylate de 2-octyle est envoyé sur une colonne de distillation C1' permettant de récupérer :
o en tête un flux 6 riche en 2-octanol qui est recyclé à la réaction,
o l'acrylate de 2-octyle pur via un soutirage latéral, et
o en pied un flux 7 riche en sous-produits lourds et contenant de l'acrylate de 2-octyle qui est envoyé sur l'évaporateur à film E à partir duquel une fraction contenant les sous-produits lourds et débarrassée de composés valorisables, est éliminée.

4. Procédé selon la revendication 1 **caractérisé en ce que** le traitement de purification du mélange réactionnel 1 comprend les sous-étapes suivantes :
- la mise en contact du mélange réactionnel 1 avec de l'eau ou un flux aqueux pour former un système biphasique constitué d'une phase aqueuse 4 comprenant le catalyseur, et d'une phase organique 5 riche en produit de réaction ;
- la séparation de ladite phase aqueuse 4 et son recyclage à la réaction ;
- la séparation de ladite phase organique 5 qui est envoyée sur une première colonne de distillation C1 permettant d'obtenir :
o en tête un flux 50 riche en produit de réaction ; et
o en pied un flux 7 riche en sous-produits lourds et contenant de l'acrylate de 2-octyle, ledit flux 7 étant envoyé sur un évaporateur à film E à partir duquel une fraction contenant les sous-produits lourds et débarrassée des composés valorisables recyclés à la colonne C1, est éliminée ;
- le flux 50 riche en produit de réaction est envoyé sur une deuxième colonne de distillation C2 permettant de récupérer :
o en tête un flux 6 riche en 2-octanol qui est recyclé à la réaction ;
o l'acrylate de 2-octyle pur via un soutirage latéral ; et
o en pied un flux riche en acrylate de 2-octyle avec des inhibiteurs de polymérisation, qui est recyclé au moins en partie par la ligne 75 au niveau de la colonne C1.

5. Procédé selon l'une quelconque des revendications précédentes **caractérisé en ce que** le catalyseur est l'acide méthane sulfonique.

6. Procédé selon l'une quelconque des revendications précédentes **caractérisé en ce que** la réaction est effectuée dans un seul réacteur, le 2-octanol étant introduit en partie directement dans le réacteur, et en partie par l'intermédiaire de la colonne de distillation surmontant le réacteur.

7. Procédé selon l'une quelconque des revendications précédentes **caractérisé en ce que** la phase aqueuse issue du décanteur est soumise à une distillation permettant de récupérer le 2-octanol présent en faible teneur avant son élimination.

8. Procédé selon l'une quelconque des revendications précédentes **caractérisé en ce que** l'évaporateur à film est un évaporateur à film tombant ou à film raclé fonctionnant dans les conditions de la première colonne de distillation.

9. Procédé selon l'une quelconque des revendications précédentes **caractérisé en ce que** l'acide acrylique est d'origine renouvelable.

## Patentansprüche

1. Verfahren zur kontinuierlichen Herstellung von 2-Octylacrylat durch Umsetzung von Acrylsäure und 2-Octanol in Gegenwart eines Veresterungskatalysators vom Typ schwefelhaltige Säure und mindestens eines Polymerisationsinhibitors, wobei das durch die Veresterungsreaktion gebildete Wasser durch Destillation in einer auf den Reaktor aufgesetzten Säule in Form einer heteroazeotropen Mischung mit dem 2-Octanol mitgeschleppt wird, welche dann nach Kondensation einer Trennung in einem Phasenscheider unterworfen wird, was eine obere organische Phase und eine untere wässrige Phase ergibt, wobei die das Reaktionsprodukt und restliche Nebenprodukte enthaltende Reaktionsmischung einer Reinigungsbehandlung durch Trennmittel unterworfen wird, wobei man einerseits reines 2-Octylacrylat, andererseits die zur Rezyklierung bestimmten nicht umgesetzten Verbindungen 2-Octanol und Acrylsäure sowie den zur Rezyklierung bestimmten Katalysator erhält, wobei das Verfahren **dadurch gekennzeichnet ist, dass**:
- die wässrige Phase aus dem Phasenscheider kontinuierlich abgezogen wird und dadurch entfernt wird;
- die organische Phase aus dem Phasenscheider am Sumpf der auf den Reaktor aufgesetzten Destillationssäule kontinuierlich zurückgeführt wird;
- die Reinigungsbehandlung der Reaktionsmischung folgende aufeinanderfolgende Schritte umfasst: (i) die Abtrennung eines katalysatorreichen Stroms, der zumindest teilweise zum Reaktionsschritt zurückgeführt wird, (ii) die Abtrennung eines an nicht umgesetztem 2-Octanol reichen Stroms, der zumindest teilweise zum Reaktionsschritt zurückgeführt wird, und (iii) die Gewinnung des gewünschten reinen 2-Octylacrylats.

2. Verfahren nach Anspruch 1, **dadurch gekennzeichnet, dass** die Reinigungsbehandlung der Reaktionsmischung 1 folgende Unterschritte umfasst:
- einen ersten Schritt der Abtrennung des Katalysators mit Hilfe eines Filmverdampfers E, wonach am Sumpf ein an Katalysator und Polymerisationsinhibitoren reicher Strom 4, der zumindest teilweise zur Reaktion zurückgeführt wird, und am Kopf ein an 2-Octylacrylat reicher Strom 5 abgetrennt wird;
- der an 2-Octylacrylat reiche Strom 5 wird einer ersten Destillationssäule C1 zugeführt, die es ermöglicht, am Kopf einen an 2-Octanol reichen Strom, der zur Reaktion zurückgeführt wird, und am Sumpf einen an 2-Octylacrylat reichen Strom 7 zu gewinnen, wobei der Strom 7 einer zweiten Destillationssäule C2 zugeführt wird, die es ermöglicht, am Kopf 8 das reine 2-Octylacrylat und am Sumpf eine Fraktion, die schwere Verunreinigungen und Polymerisationsinhibitoren enthält und entweder zur ersten Destillationssäule zurückgeführt oder entfernt wird, abzutrennen.

3. Verfahren nach Anspruch 1, **dadurch gekennzeichnet, dass** die Reinigungsbehandlung der Reaktionsmischung 1 folgende Unterschritte umfasst:
- die Flashverdampfung der Reaktionsmischung 1, die zur Trennung eines an Reaktionsprodukt reichen Stroms 5 und eines Rückstands 40 der Reaktionsmischung führt;
- das Inberührungbringen des Rückstands 40 der Reaktionsmischung unter Rühren mit Wasser oder einem wässrigen Strom zur Bildung eines Zweiphasensystems, das aus einer wässrigen Phase 4, die den Katalysator umfasst, und einer organischen Phase 45, die an schweren Nebenprodukten aus der Reaktion reich ist, besteht;
- Abtrennung der wässrigen Phase 4 und Rückführung davon zur Reaktion;
- Abtrennung der organischen Phase 45, die einem Filmverdampfer E zugeführt wird, der es ermöglicht, einen Strom verwertbarer Produkte, wie Reaktionsprodukt und nichtumgesetzte Reaktanten, abzutrennen, welcher mit dem Strom 5 gemischt wird;
- der an 2-Octylacrylat reiche Strom 7 wird einer Destillationssäule C1' zugeführt, die es ermöglicht, Folgendes zu gewinnen:
o am Kopf einen an 2-Octanol reichen Strom 6, der zur Reaktion zurückgeführt wird,
o über einen Seitenabzug das reine 2-Octyl-acrylat und
o am Sumpf einen an schweren Nebenprodukten reichen und 2-Octylacrylat enthaltenden Strom 7, der dem Filmverdampfer E zugeführt wird, aus dem eine Fraktion, die die schweren Nebenprodukte enthält und von verwertbaren Verbindungen befreit ist, entfernt wird.

4. Verfahren nach Anspruch 1, **dadurch gekennzeichnet, dass** die Reinigungsbehandlung der Reaktionsmischung 1 folgende Unterschritte umfasst:
- das Inberührungbringen der Reaktionsmischung 1 mit Wasser oder einem wässrigen Strom zur Bildung eines Zweiphasensystems, das aus einer wässrigen Phase 4, die den Katalysator umfasst, und einer organischen Phase 5, die an Reaktionsprodukt reich ist, besteht;
- Abtrennung der wässrigen Phase 4 und Rückführung davon zur Reaktion;
- Abtrennung der organischen Phase 5, die einer ersten Destillationssäule C1 zugeführt wird, die es ermöglicht, Folgendes zu gewinnen:
o am Kopf einen an Reaktionsprodukt reichen Strom 50 und
o am Sumpf einen an schweren Nebenprodukten reichen und 2-Octylacrylat enthaltenden Strom 7, der dem Filmverdampfer E zugeführt wird, aus dem eine Fraktion, die die schweren Nebenprodukte enthält und von den in die Säule C1 zurückgeführten verwertbaren Verbindungen befreit ist, entfernt wird;
- der an Reaktionsprodukt reiche Strom 50 wird einer zweiten Destillationssäule C2 zugeführt, die es ermöglicht, Folgendes zu gewinnen:
o am Kopf einen an 2-Octanol reichen Strom 6, der zur Reaktion zurückgeführt wird,
o über einen Seitenabzug das reine 2-Octylacrylat und
o am Sumpf einen an 2-Octylacrylat reichen Strom mit Polymerisationsinhibitoren, der zumindest teilweise über die Leitung 75 zur Säule C1 zurückgeführt wird.

5. Verfahren nach einem der vorhergehenden Ansprüche, **dadurch gekennzeichnet, dass** es sich bei dem Katalysator um Methansulfonsäure handelt.

6. Verfahren nach einem der vorhergehenden Ansprüche, **dadurch gekennzeichnet, dass** die Reaktion in einem einzigen Reaktor durchgeführt wird, wobei das 2-Octanol zum Teil direkt und zum Teil über die auf den Reaktor aufgesetzte Destillationssäule in den Reaktor eingetragen wird.

7. Verfahren nach einem der vorhergehenden Ansprüche, **dadurch gekennzeichnet, dass** die wässrige Phase aus dem Phasenscheider einer Destillation unterworfen wird, durch die vor ihrer Entfernung das in geringem Gehalt vorliegende 2-Octanol zurückgewonnen werden kann.

8. Verfahren nach einem der vorhergehenden Ansprüche, **dadurch gekennzeichnet, dass** es sich bei dem Filmverdampfer um einen unter den Bedingungen der ersten Destillationssäule arbeitenden Fallfilm- oder einen Wischfilmverdampfer handelt.

9. Verfahren nach einem der vorhergehenden Ansprüche, **dadurch gekennzeichnet, dass** die Acrylsäure erneuerbaren Ursprungs ist.

## Claims

1. A process for continuously producing 2-octyl acrylate by reaction of acrylic acid and of 2-octanol in the presence of an esterification catalyst of acid type comprising sulfur and of at least one polymerization inhibitor, the water formed by the esterification reaction being entrained by distillation in a column surmounting the reactor in the form of a heteroazeotropic mixture with the 2-octanol which is then subjected, after condensation, to a separation in a decanter so as to give an upper organic phase and a lower aqueous phase, the reaction mixture containing the reaction product and residual by-products being subjected to a purification treatment by separation means, in order to obtain, on the one hand, pure 2-octyl acrylate, on the other hand, the unreacted 2-octanol and acrylic acid compounds intended to be recycled, and also the catalyst intended to be recycled, said process being **characterized in that**:
- the aqueous phase derived from the decanter is continuously taken off so as to be removed;
- the organic phase derived from the decanter is continuously recycled at the bottom of the distillation column surmounting the reactor;
- the treatment for purification of the reaction mixture comprises the following consecutive steps: (i) the separation of a catalyst-rich stream which is at least partly recycled to the reaction step, (ii) the separation of a stream rich in unreacted 2-octanol which is at least partly recycled to the reaction step, and (iii) the recovery of the desired pure 2-octyl acrylate.

2. The process as claimed in claim 1, **characterized in that** the treatment for purification of the reaction mixture 1 comprises the following substeps:
- a first step of separation of the catalyst using a film evaporator E, at the end of which are separated, at the bottom, a stream 4 rich in catalyst and polymerization inhibitors, said stream 4 being at least partly recycled to the reaction, and, at the top, a stream 5 rich in 2-octyl acrylate;
- the stream 5 rich in 2-octyl acrylate is sent to a first distillation column C1 which makes it possible to recover, at the top, a stream 6 rich in 2-octanol which is recycled to the reaction and, at the bottom, a stream 7 rich in 2-octyl acrylate which is sent to a second distillation column C2 which makes it possible to separate, at the top 8, the pure 2-octyl acrylate and, at the bottom, a fraction containing heavy impurities and polymerization inhibitors which is either recycled to the first distillation column, or removed.

3. The process as claimed in claim 1, **characterized in that** the treatment for purification of the reaction mixture 1 comprises the following substeps:
- flash evaporation of the reaction mixture 1 resulting in the separation of a stream 5 rich in reaction product and of a reaction mixture residue 40;
- bringing, with stirring, the reaction mixture residue 40 into contact with water or an aqueous stream so as to form a two-phase system consisting of an aqueous phase 4 comprising the catalyst, and an organic phase 45 rich in heavy by-products resulting from the reaction;
- separation of said aqueous phase 4 and recycling thereof to the reaction;
- separation of said organic phase 45 which is sent to a film evaporator E which makes it possible to separate a stream of exploitable products, such as reaction products and unreacted reagents, which is mixed with the stream 5;
- sending the stream 5 rich in 2-octyl acrylate to a distillation column C1' which makes it possible to recover:
o at the top, a stream 6 rich in 2-octanol which is recycled to the reaction,
o the pure 2-octyl acrylate via side take-off, and
o at the bottom, a stream 7 rich in heavy by-products and containing 2-octyl acrylate which is sent to the film evaporator E from which a fraction containing the heavy by-products and from which exploitable compounds have been removed, is removed.

4. The process as claimed in claim 1, **characterized in that** the treatment for purification of the reaction mixture 1 comprises the following substeps:
- bringing the reaction mixture 1 into contact with water or an aqueous stream so as to form a two-phase system consisting of an aqueous phase 4 comprising the catalyst, and of an organic phase 5 rich in reaction product;
- separation of said aqueous phase 4 and recycling thereof to the reaction;
- separation of said organic phase 5 which is sent to a first distillation column C1 which makes it possible to obtain:
o at the top, a stream 50 rich in reaction product; and
o at the bottom, a stream 7 rich in heavy by-products and containing 2-octyl acrylate, said stream 7 being sent to a film evaporator E from which a fraction containing the heavy by-products and from which the exploitable compounds recycled to the column C1 have been removed, is removed;
- sending the stream 50 rich in reaction product to a second distillation column C2 which makes it possible to recover:
o at the top, a stream 6 rich in 2-octanol which is recycled to the reaction;
o the pure 2-octyl acrylate via a side take-off; and
o at the bottom, a stream rich in 2-octyl acrylate with polymerization inhibitors, which is at least partly recycled, via the line 75, to the column C1.

5. The process as claimed in any one of the preceding claims, **characterized in that** the catalyst is methanesulfonic acid.

6. The process as claimed in any one of the preceding claims, **characterized in that** the reaction is carried out in a single reactor, the 2-octanol being introduced partly directly into the reactor and partly by means of the distillation column surmounting the reactor.

7. The process as claimed in any one of the preceding claims, **characterized in that** the aqueous phase from the decanter is subjected to a distillation which makes it possible to recover the 2-octanol present in a small amount before it is removed.

8. The process as claimed in any one of the preceding claims, **characterized in that** the film evaporator is a falling film or wiped film evaporator operating under the conditions of the first distillation column.

9. The process as claimed in any one of the preceding claims, **characterized in that** the acrylic acid is of renewable origin.
